# EUROPEAN PATENT SPECIFICATION

(11) **EP 3 304 112 B1**
(45) Date of publication and mention of the grant of the patent: **11.10.2023**
(21) Application number: 16745417.2
(22) Date of filing: 30.05.2016
(51) Int. Cl.: G01R 33/28, G01R 33/565, A61B 90/00, A61B 90/50, A61B 34/20

(54) **SYSTEM FOR TRACKING POSITION AND ORIENTATION OF AN OBJECT IN A MAGNETIC RESONANCE (MR) APPARATUS**
SYSTEM ZUR VERFOLGUNG DER POSITION UND AUSRICHTUNG EINES OBJEKTS IN EINER MAGNETRESONANZ (MR)-VORRICHTUNG
SYSTÈME DE POURSUITE DE LA POSITION ET DE L'ORIENTATION D'UN OBJET DANS UN APPAREIL À RÉSONANCE MAGNÉTIQUE (RM)

(30) Priority: 29.05.2015 EP 15169996
(43) Date of publication of application: 11.04.2018
(73) Proprietor: Eidgenössische Technische Hochschule (ETH), 8092 Zürich (CH); Universität Zürich, 8006 Zürich (CH)
(72) Inventor: BRUNNER, David O., 8092 Zürich (CH); SCHMID, Thomas, 8092 Zürich (CH); BARMET, Christoph, 8092 Zürich (CH); PRÜSSMANN, Klaas P., 8092 Zürich (CH)
(74) Representative: Schmauder & Partner AG Patent- & Markenanwälte VSP
(86) International application number: PCT/EP2016/062200
(87) International publication number: WO 2016/193233

(56) References cited:
- US-A- 4 642 786
- US-A- 5 041 791
- US-A1- 2003 117 270
- US-A1- 2004 127 787
- US-A1- 2006 079 764
- US-A1- 2009 281 419

## Description

### Field of the Invention

The present invention generally relates to a system and to a method for tracking position and orientation of an object in a magnetic resonance (MR) apparatus and to uses of an electromagnetic position and orientation measurement device.

### Background of the invention

Magnetic resonance (MR) imaging and spectroscopy are often used for the examination of living structures such as body parts of humans or animals. Motion of the examination object - be it induced by body motion or vibration of MR-scanner parts - can rarely be fully suppressed. Moving objects (e.g. body parts) are a common issue in MR data acquisition and reconstruction, leading to erroneous object excitation, image artifacts or data misinterpretation. Accordingly, it would be desirable to track the position and/or the orientation of an object of interest in a MR imaging or spectroscopy apparatus.

US 6,879,160 (Jakab) discloses a MR scanner comprising an assembly for generating a static magnetic field having a homogeneous sample region. The MR scanner further comprises a repositionable magnetic field source for generating a spatially distinctive, i.e. inhomogeneous magnetic field. A spatial magnetic field sensor attached to the object of interest provides a spatial sensor signal that is dependent upon the position of the field sensor relative to the spatially distinctive magnetic field. This sensor signal can be used for determining the position and/or orientation of the object of interest. In a preferred embodiment, the spatially distinctive magnetic field is generated by means of magnetic field gradient generating coils of the MR scanner that are used for spatial encoding of the nuclear spins sampled by the MR scanner. A disadvantage of the arrangement described in US 6,879,160 is constituted by the fact that the signals needed for object tracking are taken from the field sensor attached to the object of interest, thus requiring appropriate signal transmission means such as signal wires or an active wireless signal transmitter to be attached to the field sensor. Both types of signal transmission means are undesirable in many practical situations.

US 4,642,786 (Hansen) discloses a method and apparatus for position and orientation measurements using a magnetic field and retransmission. The basic operating principle consists in having retransmitter means attached to the object of interest. A magnetic field resonant at a resonance frequency of the retransmitter means is generated with appropriate transmitter means; a corresponding signal emitted by the retransmitter means is received by receiver means and used for calculating the position and/or orientation of the retransmitter means. However, US 4,642,786 does not mention the integration of the tracking system into an MR imaging or spectroscopy arrangement and the required means for ensuring mutual compatibility of the two systems.

US 2004/0127787 A1 (Dimmer) discloses an implantable marker with a leadless signal transmitter compatible for use in MR devices. The marker necessarily comprises a ferromagnetic element the volume of which is limited such that when the marker is in an imaging magnetic field having a field strength of 1.5 T and a gradient of 3 T/m, the force exerted on the marker by the imaging magnetic field is not greater than gravitational force exerted on the marker. However, this requirement does not take into account nonmechanical unwanted effects caused by a ferromagnetic element placed in an MRI environment.

US 2006/0079764 A1 (/Wright) discloses systems and methods for real time tracking of targets in radiation therapy and other medical applications. The tracking concept is based on an on/off scheme wherein makers are first energized by a pulsed excitation source and subsequently interrogated during a period when the excitation is switched off. The applicability in MRI environments is mentioned by referring to the above markers with ferromagnetic elements. Such a system does not allow for continuous position tracking and, moreover, the sensitivity of the resonating markers comprising a ferromagnetic part is expected to be reduced if the device is located in a strong magnetic field.

US 2003/0117270 A1 (Dimmer) discloses a system for spatially adjustable excitation of a leadless miniature marker assembly.

US 2009/0281419 A1 (Troesken) discloses a system for determining the position of a medical instrument.

US 2014/0171784 A1 (Ooi) discloses a method for 3D motion tracking in an MRI scanner using markers containing an MR-visible sample coupled to a resonant circuit which in turn is inductively coupled to one or more receive coils of the MRI system. The tracking prinicple relies on measuring an MR signal which is spatially encoded by an appropriately selected gradient field.

### Summary of the Invention

It is thus an object of the present invention to provide an improved system for tracking position and orientation of an object in a magnetic resonance (MR) apparatus. In particular, such system shall overcome the limitations and disadvantages of presently known systems.

According to one aspect of the invention, there is provided a system for tracking position and orientation of an object in a magnetic resonance (MR) apparatus, the system comprising an MR apparatus and a tracking device for electromagnetic measurements of position and orientation,
the MR apparatus comprising:
   a) magnet means configured to generate a main magnetic field in a sample region;
   b) encoding means configured to generate encoding magnetic fields superimposed to the main magnetic field,
   c) RF transmitter means configured to generate MR radiofrequency fields;
   d) driver means configured to operate said encoding means and RF transmitter means to generate superimposed time dependent encoding fields and radiofrequency fields according to an MR sequence for forming images or spectra; and
   e) acquisition means configured to acquire an MR signal from said object; the tracking device comprising:
      a) a tracker structure that is firmly attachable to the object of which the position and orientation are to be tracked;
      b) retransmitter means firmly attached to said tracker structure, said retransmitter means having at least one retransmitter resonance frequency; and
      c) electrical circuitry means including:
         i) transmitter means configured to transmit an electromagnetic field with at least one of said retransmitter resonance frequencies;
         ii) receiver means configured to receive an electromagnetic field retransmitted by said retransmitter means; said receiver means converting said electromagnetic field into a proportional voltage;
            wherein said transmitter means and receiver means are maintained in a known positional and orientational relationship between each other, thereby defining a reference frame of said tracking device; and
         iii) calculating means configured to determine, from said proportional voltage obtained from said receiver means, a position and orientation of said retransmitter means, and concomitantly of said tracker structure, with respect to said reference frame, from which the position and orientation of said object is trackable;
wherein said retransmitter resonance frequencies are substantially different from the frequencies generated in said MR sequence; and wherein said transmitter means, retransmitter means and receiver means are electromagnetically decoupled from said RF transmitter means and encoding means, and wherein said transmitter means and said receiver means are electromagnetically decoupled from each other so as to allow uninterrupted tracking of position and orientation during operation of the MR apparatus.

Advantageous embodiments are defined in the dependent claims and in the following description.

The above defined system according to the invention, which will also be called as "tracking system", generally comprises an MR apparatus and a tracking device for electromagnetic measurements of position and orientation.

The term "object" shall be understood here as a general reference to an object of interest, which may include a human subject or an animal, a body part thereof, but also any kind of sample that may be studied or characterized by means of an MR apparatus. In an advantageous embodiment, the object is a human subject or a body region thereof. The term magnetic resonance (MR) includes nuclear magnetic resonance, but also electron spin resonance or electron paramagnetic resonance. In a particularly advantageous embodiment, however, it will refer to nuclear magnetic resonance. Accordingly, an MR apparatus will refer, in particular, to an apparatus for carrying out magnetic resonance spectroscopy, and most particularly, to an apparatus for carrying out nuclear MR imaging, henceforth abbreviated as "MRI".

Such an MR apparatus generally comprises magnet means for generating a main magnetic field in a sample region. It is generally desirable that the main magnetic field be substantially homogeneous at least within the sample region.

The MR apparatus further comprises encoding means for generating encoding magnetic fields superimposed to the main magnetic field. In particular, the encoding means can be configured as gradient coil for generating time dependent gradient magnetic fields as generally known in the field of MRI. However, the terns "encoding" shall not be limited to gradients, i.e. "encoding means" shall also include means configured for generating time dependent magnetic fields that are of higher order in space.

The MR apparatus also comprises RF transmitter means for generating MR radiofrequency fields at a predetermined magnetic resonance frequency. The latter, also denoted as Larmor frequency, is determined by the strength of the main magnetic field and by the type of MR transition observed.

Moreover, the MR apparatus comprises driver means for operating said encoding means and RF transmitter means to generate superimposed time dependent encoding fields and radiofrequency fields according to an MR sequence for forming images or spectra. In general an MR pulse sequence comprises a train of identical or similar sequence modules that are generated with a sequence repetition period T_{R} between each pair of successive sequence modules. Typically it is one of many known sequences used for MR spectroscopy or MRI measurements depending on the specific purpose.

Finally, the MR apparatus comprises acquisition means for acquiring an MR signal from said object. Such acquisition means may comprise various types of coils or coil array for detection of the MR signal emitted by the object of interest following MR excitation thereof. The acquisition means may be mounted to the object or they may be fixed in any other reference system, e.g. to a part of the MR apparatus.

The tracking device is configured for carrying out electromagnetic measurements of position and orientation. It comprises a tracker structure that is firmly attachable to the object of which the position and orientation are to be tracked. Its principal purpose is to establish a fixed spatial relation between retransmitter means and object of interest. Accordingly, the mechanical stability or firmness of the tracker structure itself and of the respective attachment means will have to conform with the required accuracy of the tracking method.

In order to exhibit control over the nuclear magnetization for excitation, contrast formation, spinfiltering but also spatial encoding, an MRI scanner produces magnetic fields in different frequency bands. The magnetic fields used for inducing nutations in a spin species are located in a narrow band (approx. 1 MHz) around the Larmor frequency of the particular nucleus at the given field strength, i.e. 64 MHz for protons at 1.5 T. The fields employed for spatial encoding (e.g. gradients) by inducing spatially defined phase relations are switched at frequencies significantly below 50 kHz. Since typically switched mode amplifiers are employed for driving these coils, fields at the employed switching frequency (typically between 10 kHz and 150 kHz) and even at higher harmonics thereof are present. Furthermore the Faraday cages employed to shield the MRI scanner from interferences from the environment are typically made of copper foils. Consequently, their shielding capability is limited below 100 kHz. Therefore the frequency window above the switching frequency of the gradient and shim amplifiers and below the Larmor frequency is unoccupied. On the other hand an inductive tracking device is preferably operated at frequencies at which the influence of dielectric load introduced by the subject on the field employed for tracking is low. For these reasons the tracking device is preferably selected to operate in a frequency window between 500 kHz and 20 MHz.

By virtue of the above described features, i.e.
- retransmitter resonance frequencies being substantially different from the frequencies generated in the MR sequence;
- transmitter means, retransmitter means and receiver means being electromagnetically decoupled from the RF transmitter means and encoding means, and
- transmitter means and receiver means being electromagnetically decoupled from each other,
it is possible to operate the tracking device in a substantially continuous manner, i.e. without being forced to interrupt the tracking operation in order to avoid interactions from other components of the system.

In principle, the absolute position and orientation of the object of interest could be determined from the absolute position and orientation of the tracker structure that is firmly attached to the object. However, there are many situations in which it is sufficient to know any movements and re-orientations that have undergone in relation to an initial position and orienation at a reference time point. This could be e.g. the start of an MR scan. The determination of such relative changes of position and orientation will be understood to fall under the term "tracking". This in turn means that in certain practical situations it will be sufficient to determine the position and orientation of the tracker structure in order to track the concomitant relative movement and reorientation of the object of interest. This type of information is sufficient, e.g for adjusting an MR image reconstruction or the forthcoming modules of a running MR sequence.

According to one embodiment (claim 2), the tracker structure is configured for attachment to a head region of a patient. In particular (claim 3), the tracker structure is selected from the group consisting of ear muff, ear plug, helmet, headgear, tooth attachment, jaw attachment, nose clip, wrist attachment, ankle attachment, stereotactic frame, splint and prosthesis.

An attachment to a tooth or to an upper jaw portion provides a particularly firm positioning, thus substantially increasing the tracking accuracy.

The retransmitter means shall have at least one retransmitter resonance frequency. In many situations it may be preferable to have a plurality of distinct resonance frequencies. They should be sufficiently distinct to allow for selective excitation and detection, but preferably close enough to allow using the same technology for the excitation and detection of all the resonance frequencies of one retransmitter means.

The transmitter means and the receiver means of the tracking device shall be electromagnetically decoupled from each other. This is important because at the location of a receiver the electromagnetic field originating from a transmitter will be substantially stronger than that originating from a retransmitter and would thus make detection of the latter field very difficult. Such decoupling may be achieved by
i) geometric provisions, i.e. relative orientation, counter-loops and overlapping loops;
ii) decoupling networks between transmitters and receivers, preferably configured as adjustable networks;
iii) active compensation via signal subtraction, e.g. by means of operational amplifiers.

Alternatively or additionally, an efficient decoupling is achieved by configuring the retransmitters to emit an electromagnetic field with a frequency that differs from the basic resonance frequency generated by the transmitters. The receivers can then be configured to operate at this different frequency and to be essentially insensitive for any electromagnetic field contributions at the basic resonance frequency. For example, this may be achieved by providing the retransmitters with a diode element acting as a frequency doubler. Accordingly, the retransmitter will emit at the double frequency as compared to the transmitter, and the receiver can be configured for selective detection of this double frequency.

The tracking device further comprises electrical circuitry means including i) transmitter means for transmitting an electromagnetic field at - at least - one of said retransmitter resonance frequencies, and ii) receiver means for receiving an electromagnetic field retransmitted by said retransmitter means and for converting said received electromagnetic field into a proportional voltage.

The transmitter means and the receiver means shall be maintained in a known positional and orientational relationship between each other, thereby defining a reference frame of said tracking device. In many practical situations the transmitter means and the receiver means will both be attached to a common rigid structure, whereby the positional and orientational relationship between each other will be constant in time. However, it is also possible to implement a time dependent but nonetheless known positional and orientational relationship, e.g. by appropriately driven guiding means, such as in a fixedarm rotating or in a linear rail-guided sliding system or in even more complex systems.

According to one embodiment (claim 4), the transmitter means and the receiver means are firmly attached to a structural component of the MR apparatus. The structural component may be a completely space-fixed part, such as a support beam of the apparatus. However, it may also be a part that is movable in a controlled manner and may be brought into a desired positional relationship with a part of the MR apparatus, such as a slidable patient bed that can be moved into and out of a MRI cavity. In certain applications it might be considered as beneficial to integrate the transmitting function and/or the receiving function of the tracking device at least partly in the same electric structures as used for transmitting the excitation pulses for the MR and/or receiving the MR signals.

In certain embodiments, some of the receiver means are configured as single loop receivers. In other embodiments, some of the receiver means are configured as gradiometer loop receivers (claim 5). Gradiometers generally yield less signal per area, but they provide stronger relative signal variations caused by movements of the retransmitter means. Moreover, gradiometers suffer less from signal pickup from the MR system.

Any parts of the tracking device that will be immersed in the main magnetic field at some point of the operation of the tracking device shall be made of materials that are substantially non-magnetic. Otherwise, the tracking device would experience highly undesirable torques and forces in the very strong magnetic fields used in any MR apparatus. Particularly the structures that come close to the sampled (in MR spectroscopy) or imaged (in MR imaging) portion of the object shall be made of materials that are at mostly weakly magnetic. Otherwise, the tracking device would lead to spectrum or image degradation (image warping or blurring, signal de-phasing, etc.). Parts and components residing in close vicinity to the sampled or imaged portion of the subject have to fulfill this requirement the most since the secondary magnetic field produced by such magnetic components would distort the uniformity of the main magnetic field which is required for proper MR signal acquisition.

However, in order to render the part of the device entering the bore of the MRI system and in particular devices residing in close proximity or in the field of view of the MRI scan to be MR compatible with respect to the static magnetic field produced by the scanner, not only the mechanical forces have to be considered. The magnetic field produced by ferro-, ferri or strongly or superparamagnetic materials typically distorts the magnetic field in the scanner and hampers thereby the imaging and spectroscopic acquisition. The magnetization and amount of material that is toleratable under this point of view is typically much lower than with respect to the exhibited mechanical forces. Furthermore, magnetic materials employed in electronic devices to boost the inductance of coils, block sheath currents or shield magnetic fields become ineffective when immersed in the main magnetic field. This because the magnetic material is partially, in the case of ferrites typically fully saturated by the external field. This effect renders the inductance and the Q RF coils with ferrite or iron powder cores and shields very low once entering the main magnetic field, sometimes even irreversibly.

Moreover, the one or several retransmitter resonance frequencies used according to the invention shall be substantially different from the frequencies generated by the driver means operated according to the selected MR sequence. In other words, the resonance frequencies shall be: i) higher than the low frequency ("acoustic") frequencies generated by the gradient coils or any other encoding means, and ii) lower than the MR radiofrequencies used for the MR measurement.

Therefore, according to an advantageous embodiment (claim 6), the retransmitter resonance frequencies are in the range of 50 kHz to 20 MHz, preferably in the range of 500 kHz to 5 MHz.

When used for human subjects, the operating frequency of the transmitters shall be low enough to meet the requirements for the specific absorption rate (SAR) in tissue.

The electromagnetic fields at the above mentioned frequencies penetrate live tissue innocuously. Therefore the reflectors can be positioned at locations where optical markers would be occluded to a camera, e.g by hair or when placed into an ear, on a tooth, upper jaw or would be occluded by the applied receiver coil.

Furthermore, the transmitter means, retransmitter means and receiver means shall be electromagnetically decoupled from the MR-RF transmitter means and encoding means. The term "electromagnetically decoupled" shall be understood to imply the absence of any substantial electromagnetic interaction at least in frequency bands occupied by the operation of the tracking device and of the MR device (i.e. the tracking device shall be substantially decoupled from the gradient and shim coils in the acoustic frequency range as well as from MR-RF coils at the Larmor frequency). Accordingly, any coil elements of the transmitter, retransmitter and receiver means shall be transparent at the frequencies of encoding means but also at the MR radiofrequency.

This can be achieved with basically known means, which generally comprise electronic means such as frequency filters, lumped or distributed trapping circuits and compensating circuitry, but may also comprise geometric means regarding the relative arrangement of components. One goal to be achieved by such decoupling relates to eddy currents that may be produced in various parts of the MR apparatus and by the tracking device.

According to an advantageous embodiment (claim 7), the transmitter means are configured as a transmitter loop with a transmitter loop size, and said receiver means are configured as receiver loops having a receiver loop size that is substantially smaller than the transmitter loop size,. Such a configuration is particularly useful when the retransmitters are located in a region displaced from the transmitter loop plane by up to about the loop size (i.e. the diameter in case of a circular loop) and within the perimeter of the transmitter loop. Typically, a transmitter loop diameter of about 30 to about 100 mm, particularly from about 40 to about 80 mm and more particularly from about 50 to about 60 mm is useful. Typically, the number of receivers ranges from four to ten. Advantageously, the receiver loops are positioned within said transmitter loop (claim 8).

In an exemplary embodiment, one transmitter with a loop diameter of about 60 mm and seven receivers with loop diameter of about 14 mm positioned within the transmitter loop are arranged on a planar board element. Six of the receivers are arranged hexagonally and are configured as magnetic gradiometers to measure a magnetic field gradient in radial direction. The seventh receiver is arranged centrally and is configured as an ordinary electromagnetic field detector.

According to an advantageous embodiment (claim 9), the retransmitter means comprise at least one resonant loop element. This simple configuration allows for a very compact and simple construction, which is important in view of several uses at or within a human subject. A high quality factor ("Q-factor") is generally desirable in order to have a narrowbanded resonance behavior and an efficient retransmission. Advantageously, the tracking device comprises several loop type retransmitters having distinct resonance frequencies and having different loop planes. Unwanted interactions between retransmitters are suppressed by using a partially overlapping loop configuration. Typically, the size of the retransmitter loops (i.e. the diameter in case of a circular loop) is in the range of about 10 to 20 mm. Larger loops generally provide more signal but smaller spatial resolution.

The position and orientation of the tracker structure relative to the object can be determined by MR image data that contains the object (or portions thereof such as slices or other subvolumes) as well as signal from one or several MR active markers that are attached to or contained in the tracker structure (claim 10). These markers must be made and arranged in such manner that the position and orientation of the tracking structure can be unambiguously determined. This is provided, e.g., by a number of spherical markers suitably arranged in space, or by a marker of irregular shape. The marker can contain a compound that is MR-active at the same frequency as the object of interest, which would be the case when a water-based marker is used in a proton MR scan. However, the marker can also contain other nuclei such as fluorine, carbon, phosphorous, sodium, deuterium, etc. that are NMR-active at a different frequency. In this case the images from the object and the marker - obtained at the two frequencies - can be superimposed to determine the relative position and orientation of the two entities, i.e. the tracker structure and the object of interest. Relaxation, chemical shift and susceptibility properties of the marker compound can be adapted by the addition of suitable compounds (such a copper sulfate in a water marker or gadolinium FOD in a hexafluorobenzene marker, etc.).

According to another aspect of the invention, there is provided a method of tracking position and orientation of an object in a magnetic resonance (MR) apparatus by means of a tracking system as defined above, the method comprising the steps of:
a) firmly attaching said tracker structure to the object of which the position and orientation are to be tracked;
b) operating said transmitter means to transmit an electromagnetic field with at least one of said retransmitter resonance frequencies;
c) receiving an instant electromagnetic field retransmitted by said retransmitter means; and
d) calculating an instant position and orientation of said retransmitter means, and concomitantly of said tracker structure, with respect to said reference frame, from which the position and orientation of said object is trackable.

It will be understood that step d) will generally be based on numeric computations requiring substantial computational power, particularly in view of time requirements of a real time tracking.

The basic theoretical framework for these calculations has been disclosed in US 4,642,786 (Hansen).

According to an advantageous embodiment (claim 12 ) the MR apparatus is an MR imaging apparatus and the tracker structure is provided with at least one MR active marker, and the method further comprises the steps of acquiring MR image data of the object and of the MR active markers and subsequently determining therefrom a relative position and/or orientation between said object and said tracker structure.

The above methods have the important advantage of not requiring a free line of sight for the tracking process, in contrast with presently used methods relying on optical devices. It should be pointed out that any measurement of position and orientation termed here as "tracking" shall be in relation to the above defined reference frame, but not necessarily to a space-fixed ("laboratory") frame.

According to an advantageous embodiment (claim 13), the tracking device is used for at least one of the following:
- issuing a warning that a change of position and/or orientation of the object has occurred exceeding a predefined threshold;
- rejecting and optionally repeating the acquisition of a set of MR signals from said object when a change of position and/or orientation of the object exceeding a predefined threshold has occurred;
- using position and orientation information for MR image reconstruction;
- applying a correction for motion artifacts in MR image reconstruction;
- updating the MR sequence;
- extraction of physiological information such as on states (tremor, epileptic seizure) and parameters (breathing, heartbeat, muscle tension, nervousness);
- combined analysis of physiological information and MR data.

According to another advantageous embodiment (claim 14), the object is a human subject and the tracker structure is attached to a location of said subject selected from the group of: ear/auditory canal; skull; tooth; jaw; nose; wrist; and ankle.

### Further remarks concerning hardware

It has been found that the receivers must provide a signal-to-noise ratio ("SNR") of more than 40 dB (scaled to the maximum of all signals acquired from each particular position) for determining the position with an accuracy of less than 0.1 mm and 1°. Therefore the employed analog-to-digital converters ("ADCs") must provide an SNR of 40 dB, preferably 60dB, on the bandwidth where the motion is tracked. This explicitly includes the conversion gain from resampling from the higher ADC framing rate to the lower motion framing rate. In fact it will be often preferred to use ADCs that allow to simultaneously sample the frequency span covered by all employed frequencies of the tracker. Since the phase relations of the signals between the transmitter and the receivers are of critical relevance, it is preferred to use a common timing, clock or reference signal for driving the signal sources in the transmitter as well as the demodulators and or the sampling clocks of the ADCs. This would also inherently cancel out large portions of timing jitter present in the reference clock signal from the received coupling signals.

For the signal sources oscillators or direct sampling DACs can be employed. Thereby the dynamic range of the signal source must be such that the output noise level seen through the direct coupling from the transmitter to the receiver does not lower the noise floor of the measurement of the retransmitter signals.

The frequency of operation of the retransmitters is driven by following three considerations: firstly, the acquisition band has to be high enough in order to be not confounded by the action of the MRI gradient switching, it shall furthermore be clear of spurs and interactions with the high power transmission pulses of the MRI systems. And lastly the individual frequencies of operation have to spaced sufficiently (delta f>f/Q) such that the couplings to the receivers by each re-transmitter in close vicinity to each other are highly distinctive.

In a preferred embodiment the receivers and the transmission signal generator of the motion tracking device are placed in close vicinity of the MRI coil since routing conductive cables out of the bore involves problems with RF compliance and usability. It is therefore preferred to transfer previously digitized data over RF or optical links.

Further the employed signal sources, ADCs, digital signal transmission and active electronics must provide very low noise and spur emissions at the employed MR frequencies. In preferred embodiments the employed integer multiples or fractions of the clock frequencies do not fall into the acquisition bands of the MRI scanner. Further MRI compatible RF shielding components as disclosed in EP 2708908 A1 can be employed to lower the interference of the tracking device to the scanner.

### Tracking algorithm: determining retransmitter position from the received signals

In a first step the signals acquired by the ADCs are filtered to isolate the working frequencies at which at least one of the retransmitters is resonant. This can either be done by cascaded integral comb filters (CIC), finite impulse response filters (FIR) or infinite impulse response filters (IIR). An implementation using a sliding time windowed FFT for isolating the individual frequencies at which the retransmitters respond can be efficient if the number of retransmitters is large. Thereby the signals emitted by the retransmitters can be distinguished from signals directly coupled from the transmitters to the receivers by an additional 90° phase shift occurring on resonance, which correspondingly gives an appropriate and fixed phase relation among the transmitter and receiver, to the quadrature component (with respect to the transmitted signal phase at each employed frequency) of the I/Q demodulated signals. Further, direct coupling components and their phase relation can be calibrated when the retransmitter is not present or is positioned far away.

Once the signals of each retransmitter are isolated, the position of the re-transmitter can be found by fitting all the obtained signals to a signal model which describes the dependence of the received signal on the position. In typically proposed methods (see e.g. US 4,642,786), this model is based on ideal dipoles for transmitting and receiving. However, it is expected that such models are not sufficient for many practical situations. Further measuring a full calibration table for describing the signal behavior by interpolation to a measured look up table was found to be unfeasible because of the large amount of required measurement points, which would render the calibration procedure unfeasible. Therefore, a hybrid approach of analytically derived models based on the actual distribution of the conducting materials which will be calibrated by measuring model inherent parameters is proposed. Such parameters preferably represent the effective coupling strength among the retransmitters and the transmitter and receivers, the effective dipolar, quadrupolar and higher order moments of the electric structures, eddy current estimates of conductive structures nearby, effective diameters of the involved loop structures, effective positions and others.

This enhanced signal model is then evaluated in real time or can be precalculated partially or in total and included in a look-up table.

The obtained signal vector containing the retransmitter signals received at each frequency and from each receiver are then fitted to said signal model by use of an iterative search algorithm such as Gauss-Newton, Gauss-Seidel, Simplex search algorithm or similar. It was found to be especially beneficial to implement the involved matrix inverse operation by iterative algorithms such as conjugate gradients, especially when operating on FPGAs (field programmable gate array) and DSPs (digital signal processor). Thereby the initial starting point of the scan series can be found by finding the point in the look-up table with minimum norm difference to the present signal vector. Once the device is tracking the position of the object, the starting point of the iterations can be estimated to be in close vicinity of the true position by using the information obtained from the previously acquired kinetic state of the object and applying the known constraints on the changes of said kinetic state such as the objects maximum linear and angular speed, jerk and bandwidth limitation. These limitations can however not only be used to estimate the position of a next measurement point, but also to regularize the obtained trajectory found by the tracking device.

The tracking device will then preferably make the information on the sample motion available to the MRI scanner's sequence controller and/or reconstructor by a low latency buffer or queue, preferably over IP networks working at least in the same subnet. In its most effective implementation the scanner will update the acquisition with a very short latency with respect to the occurring motion (<20 ms). Therefore the mentioned processing steps have to be performed using means for real-time computing, preferably using dedicated processor pipe-lines and cores.

The rate at which the positions have to be calculated from the acquired signals needs to be shorter than dictated by the expected bandwidth of the motion, but it is at the same time also dictated by the maximum speed of the object. The object moving over the receivers changes the coupling, which is, however, only represented in the signal model to be stationary. Therefore the averaging properties of the signals in the previously mentioned filters have to be such that the signals retrieved from the moving subject are still representative for the signal model. This is no longer the case if the motion during an averaging time of the input filters runs over significantly non-linear signal responses.

If an arrangement with several retransmitters having a fixed position relative to each other during the data acquisition is used (for instance one in each ear channel), their fixed relative position can be used to regularize the problem of position tracking.

The obtained position data can then be used in various ways in order to enhance the MRI scanning and image quality. Firstly, the correct placement of the patient in the MRI scanner can be checked if the retransmitters are mounted on a previously known anatomic structure (ear channel, teeth). Standard protocols could start align scouting or also final scans along these markers. Further the system could warn the operator that the subject moved during a scan or a scan session. Further, the scanner can reject the image acquisition or parts thereof and could optionally reacquire said portions of the image. Further the scanner could incorporate the implications of the motion when reconstructing the received data. Thereby two different classes of corrections can be taken into consideration: First the effect of the motion on the spatial encoding and shimming. Since the gradient, shim and most often the receive coils are fixed with respect to the laboratory system in which the subject moves, the encoding effect of the encoding moves with subject motion in the frame of reference of the subject. This means that the effect of gradient encoding, shim and non-linear encoding fields, as well as coil sensitivities have to be transposed accordingly in order to reconstruct the obtained data. The second class concerns the effect of motion on the resulting contrast formation in the sequence which can be corrected in many cases using the known trajectory of the sample. Especially the sensitivity to velocity that is exhibited by most sequences represents an additional source of errors beside the influence of the motion on the image encoding itself. Thereby first order gradient moments (and higher order moments correspondingly for acceleration, jerk etc.) encode a phase and in the case of strong moments (as used for diffusion encoding) an amplitude reduction by dephasing onto the velocity of the subject. In most cases these additional intra-shot effects of bulk motions are unwanted and can deteriorate the resulting images or add errors to the resulting images (e.g. diffusion weighted and velocity encoded scans, multishot echo planar imaging and spiral acquisition etc.).

A further approach of correcting the subject motion during scanning is achieved by updating the acquisition parameters such as the orientation and strength of the involved gradient pulses, the frequency and bandwidth of the applied RF pulses, currents in the shim coils and even multi-channel transmit pulses to the new or prospective subject position gained by the tracking device in real time. For this purpose the position data is handed over with low latency (50 ms) to the scanner performing the updates.

A further application of the present invention lies in harvesting physiological data on the subject based on the characteristics of its motion. By this, emotional states, tremors, seizures, sleep etc. could be detected and correlated to the obtained images.

### Brief description of the drawings

The above mentioned and other features and objects of this invention and the manner of achieving them will become more apparent and this invention itself will be better understood by reference to the following description of various embodiments of this invention taken in conjunction with the accompanying drawings, wherein:
- Fig. 1: shows parts of a tracking device and an object of interest, in a schematic perspective view;
- Fig. 2: shows a photographic reproduction of a partially assembled tracking device;
- Fig. 3: shows an embodiment of a tracking device in a schematic perspective view; and
- Fig. 4: shows the standard deviation of determined position (x,y,z) in mm and orientation (α, β, γ) in degrees vs. inverse SNR of the peak signal for the specific position and orientation indicated.

### Detailed description of the invention

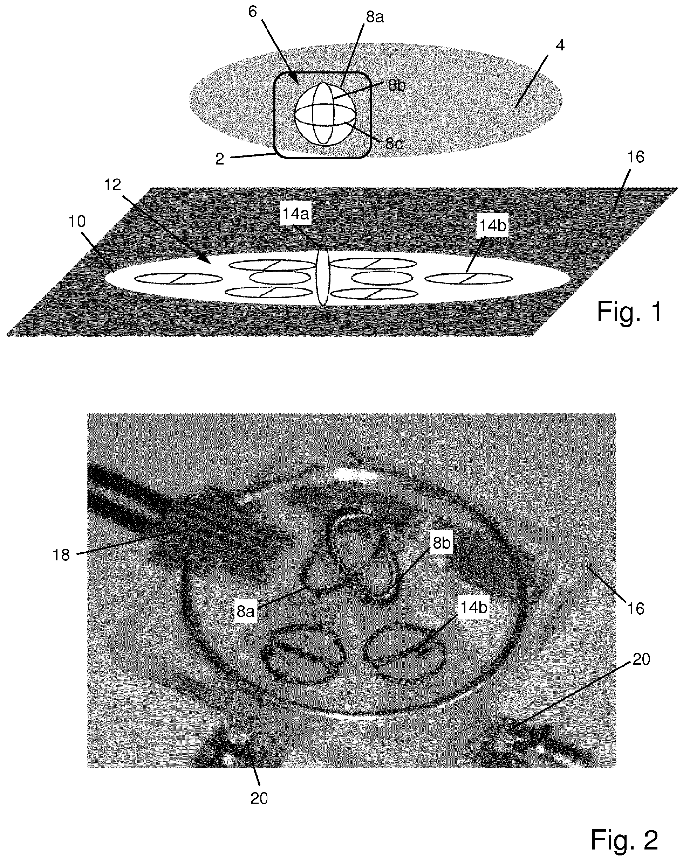

The tracking device for electromagnetic measurements of position and orientation partially shown in Fig. 1 comprises a tracker structure 2 that is firmly attachable to an object 4 of which the position and orientation are to be measured. The tracker structure comprises retransmitter means 6 firmly attached thereto. In the example shown, the retransmitter means comprise three resonance loops 8a, 8b, 8c which are arranged, for example, substantially orthogonal to each other. Advantageously, each one of the three resonance loops has at least one retransmitter resonance frequency, and these resonance frequencies are mutually different. The tracking device further comprises electrical circuitry means including transmitter means 10 for transmitting an electromagnetic field with at least one of the retransmitter resonance frequencies. In the example shown, the transmitter means are configured as a substantially circular outer loop of the tracking device. Moreover, the tracking device comprises receiver means 12 for receiving an electromagnetic field retransmitted by the resonance loops 6a, 6b, 6c. The receiver means 12 convert the received electromagnetic field into a proportional voltage which is then processed by suitable processor means not shown here. In the example shown, some of the receiver means are configured as simple loop receivers 14a and others are configured as gradiometer loop receivers 14b. The transmitter means 10 and receiver means 12 are maintained in a known positional and orientational relationship between each other by attachment to a base plate 16. As shown schematically in Fig. 1, most of the loop receivers are arranged in a plane defined by the base plate 16, whereas one loop receiver is oriented substantially orthogonal to the plane in the example shown here. It will be understood that the mounted arrangement shown here serves to define a reference frame of the tracking device.

Fig. 2 shows a partially assembled tracking device including some of the components shown in Fig. 1, which are denoted by the same reference numerals as in Fig. 1. In the assembly of Fig. 2 the retransmitter means comprise just two resonance loops 8a and 8b and two gradiometer loop receivers 14b. Also shown in Fig. 2 are a transmitter filtering element 18 and two receiver filtering elements 20.

### Example 1: Tracking device

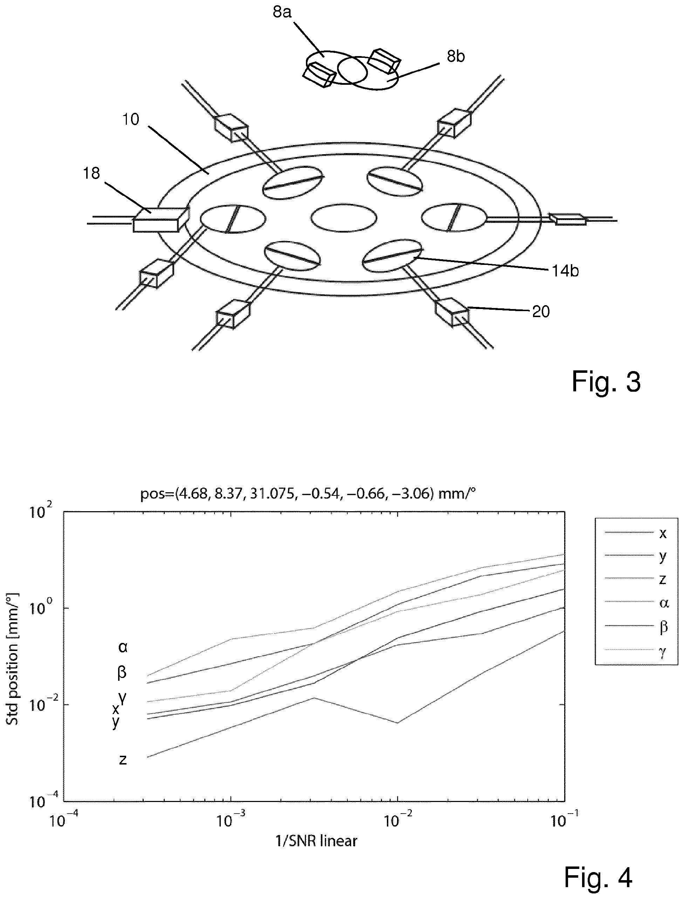

The tracking device schematically shown in Fig. 3 is configured for use in a MR imaging apparatus according to the following specifications.

### Operational signal:

- multiple low noise and low distortion sine waveforms between 1 MHz and 10 MHz (matched to the reflector resonance frequencies).

### Transmitter:

- 3-Turn loop (diameter 60mm) with counter-loop to minimize direct coupling to receiver coils. Construction on a printed circuit board (PCB) with 0.3mm line width
- Filter block at MRI-Frequency to prevent distortions of the MRI-Field. The filter must be transparent at operational frequencies
- Multi-Sine signal generator with high impedance AC current source output. This prevents current flow due to induced voltages from MRI gradient fields
- The output current (in the range up to 100mA) is determined by the coil inductance and the operating frequency. At lower frequencies the current will be higher to counteract the reduced sensitivity at lower frequencies.
- The power can be automatically adjusted to reduce the required receiver dynamic range for different reflector distances.

### Receivers:

- Magnetic gradiometers with 14mm diameter built with 8-turn copper wire (wirediameter 0.13mm with 40µm P155 isolation).
- 6 gradiometers are arranged in the transmitter plane circularly around the transmitter center with 17mm distance from the center and 60° spacing. The gradiometers are aligned to measure the operational field gradient in radial direction from the center. A simple loop receiver coil with the same dimensions is placed in the center.
- A filter element furthermore reduces the RF-currents in the receiver coil and thus the interaction with the MRI-frequency
- A high impedance preamplifier prevents current flow from induced gradient fields and thus the interaction with the MRI-gradients. It also reduces the currents at the operating frequencies and the cross-coupling between receivers.

### Reflectors (retransmitters):

- Two resonant reflector coils pick up the signal from the transmission coil. The high Q-factor (40-70) of the reflectors insures high current flows in the reflector.
- The relative positions of the reflectors are selected in such manner that the reflectors are decoupled among each other.
- A capacitor/filter block produces resonance at the operating frequency and blocks current at MRI-frequency
- The reflectors have a diameter of 16mm and are built with 8 to 16 turns of 0.2mm copper-wire with 50µm P155 isolation. The capacitors are between 220pF and 3nF and the resonance frequencies between 1.1MHz and 7.2MHz

### Specifications:

Defining a reference frame with its origin at the center of the transmitter loops with the x and y axis in the plane defined by the PCB board and the z axis perpendicular to it and pointing towards the reflectors, the following ranges were found useful for tracking. A sampling frequency of > 10Hz was achieved.
Object position: x, y from -30 to + 30 mm, z from +3 to + 30mm
Object orientation angle (yaw,pitch,roll): from -20 to +20°

### Example 2: Expected precision

The results of a numeric evaluation of the achievable tracking accuracy are shown in Fig. 4. They are represented as the precision (expressed in terms of its standard deviation) in determining the coordinates (x,y,z) in mm units and orientation angle (α, β, γ) in degrees as a function of the SNR of the obtained signal from the retransmitter (actually plotted as the inverse of the SNR of the peak signal) for a specific retransmitter position (x=4.68mm, y=8.37mm, z=31.075mm) and orientation (α=-0.54°, β=-0.66°, γ=3.06°). These results indicate that an SNR of about 100 will be sufficient to determine the position to better than 100 µm and the orientation to better than 1°.

## Claims

1. A system for tracking position and orientation of an object in a magnetic resonance (MR) apparatus, the system comprising an MR apparatus and a tracking device for electromagnetic measurements of position and orientation,
the MR apparatus comprising:
a) magnet means configured to generate a main magnetic field in a sample region;
b) encoding means configured to generate encoding magnetic fields superimposed to the main magnetic field,
c) RF transmitter means configured to generate MR radiofrequency fields;
d) driver means configured to operate said encoding means and RF transmitter means to generate superimposed time dependent encoding fields and radiofrequency fields according to an MR sequence for forming images or spectra; and
e) acquisition means configured to acquire an MR signal from said object;
the tracking device comprising:
a) a tracker structure (2) that is firmly attachable to the object (4) of which the position and orientation are to be tracked;
b) retransmitter means (6;8a,8b,8c) firmly attached to said tracker structure, said retransmitter means having at least one retransmitter resonance frequency; and
c) electrical circuitry means including:
i) transmitter means (10) configured to transmit an electromagnetic field with at least one of said retransmitter resonance frequencies;
ii) receiver means (12;14a,14b) configured to receive an electromagnetic field retransmitted by said retransmitter means; said receiver means converting said electromagnetic field into a proportional voltage;
**characterized in that**:
said transmitter means and receiver means are maintained in a known positional and orientational relationship between each other, thereby defining a reference frame of said tracking device; and
said electrical circuitry means further including
iii) calculating means configured to determine, from said proportional voltage obtained from said receiver means, a position and orientation of said retransmitter means, and concomitantly of said tracker structure, with respect to said reference frame, from which the position and orientation of said object is trackable;
said retransmitter resonance frequencies being substantially different from the frequencies generated in said MR sequence;
and said transmitter means, retransmitter means and receiver means being electromagnetically decoupled from said RF transmitter means and encoding means, and said transmitter means and said receiver means being electromagnetically decoupled from each other
so as to allow uninterrupted tracking of position and orientation during operation of the MR apparatus.

2. The tracking system according to claim 1, wherein said tracker structure is configured for attachment to a head region of a patient, whereby a fixed position of the retransmitter means in relation to said head region is established.

3. The tracking system according to claim 2, wherein said tracker structure is selected from the group consisting of ear muff, ear plug, helmet, headgear, tooth attachment, jaw attachment, nose clip, wrist attachment, ankle attachment, stereotactic frame, splint and prosthesis.

4. The tracking system according to one of claims 1 to 3, wherein said transmitter means and said receiver means are firmly attached to a structural component of said MR apparatus.

5. The tracking system according to one of claims 1 to 4, wherein said receiver means are configured as gradiometer loop receiver.

6. The tracking system according to one of claims 1 to 5, wherein said retransmitter resonance frequencies are in the range of 50 kHz to 20 MHz, preferably in the range of 500 kHz to 5 MHz.

7. The tracking system according to one of claims 1 to 6, wherein said transmitter means are configured as a transmitter loop with a transmitter loop size, and wherein said receiver means are configured as receiver loops having a receiver loop size that is substantially smaller than the transmitter loop size.

8. The tracking system according to claim 7, wherein the receiver loops are positioned within said transmitter loop.

9. The tracking system according to one of claims 1 to 8, wherein said retransmitter means comprise at least one resonant loop element.

10. The tracking system according to one of claims 1 to 9, wherein the MR apparatus is an MR imaging apparatus, wherein said tracker structure comprises at least one MR active marker for establishing the relative position and orientation of the object from MR imaging data comprising the object and the MR active marker.

11. A method of tracking position and orientation of an object in a tracking system according to one of claims 1 to 9, the method comprising the steps of:
a) firmly attaching said tracker structure to the object of which the position and orientation are to be tracked;
b) operating said transmitter means to transmit an electromagnetic field with at least one of said retransmitter resonance frequencies;
c) receiving an instant electromagnetic field retransmitted by said retransmitter means; and
d) calculating an instant position and orientation of said retransmitter means, and concomitantly of said tracker structure, with respect to said reference frame, from which the position and orientation of said object is trackable.

12. The method according to claim 11, wherein said MR apparatus is an MR imaging apparatus, and wherein said tracker structure comprises at least one MR active marker for establishing the relative position and orientation of the object from MR imaging data comprising the object and the MR active marker, further comprising the steps of acquiring said MR image data of the object and of the MR active marker or markers, respectively, and subsequently determining therefrom a relative position and/or orientation between said object and said tracker structure.

13. The method according to claim 11 or 12, further comprising a step selected from the group consisting of:
- issuing a warning that a change of position and/or orientation of the object has occurred exceeding a predefined threshold;
- rejecting and optionally repeating the acquisition of a set of MR signals from said object when a change of position and/or orientation of the object exceeding a predefined threshold has occurred;
- using position and orientation information for MR image reconstruction;
- applying a correction for motion artifacts in MR image reconstruction;
- updating the MR sequence;
- extraction of physiological information such as on states, e.g. tremor or epileptic seizure, and parameters, e.g. concerning breathing, heartbeat, muscle tension or nervousness;
- combined analysis of physiological information and MR data.

14. The method according to one of claims 11 to 13, wherein said object is a human subject and wherein said tracker structure is attached to a location of said subject selected from the group of: inner ear, skull, tooth, jaw, nose, wrist, and ankle.

## Patentansprüche

1. System zum Tracking der Position und Orientierung eines Objekts in einer Magnetresonanz-(MR)-Vorrichtung, wobei das System eine MR-Vorrichtung und eine Trackingvorrichtung für elektromagnetische Messungen der Position und Orientierung umfasst,
wobei die MR-Vorrichtung umfasst:
a) Magnetmittel, die zum Erzeugen eines Hauptmagnetfeldes in einer Probenregion konfiguriert sind;
b) Kodiermittel, die zum Erzeugen von Kodiermagnetfelder, die dem Hauptmagnetfeld überlagert sind, konfiguriert sind,
c) RF-Transmittermittel, die zum Erzeugen von Radiofrequenzfeldern konfiguriert sind;
d) Treibermittel, die zum Betreiben der Kodiermittel konfiguriert sind, sowie RF-Transmittermittel zum Erzeugen von überlagerten zeitabhängigen Kodierfeldern und Radiofrequenzfeldern gemäss einer MR-Sequenz zur Erzeugung von Bildern oder Spektren; und
e) Erfassungsmittel, die zum Erfassen eines MR-Signals von besagtem Objekt konfiguriert sind;
wobei die Trackingvorrichtung umfasst:
a) eine Trackerstruktur (2), die fest an dem Objekt (4), dessen Position und Orientierung verfolgt werden soll, angebracht werden kann;
b) Retransmittermittel (6;8a,8b,8c), die fest mit der Trackerstruktur verbunden sind, wobei die besagten Retransmittermittel zumindest eine Retransmitter-Resonanzfrequenz aufweisen; und
c) elektrische Schaltungsmittel einschliesslich:
i) Transmittermittel (10), die zum Transmittieren eines elektromagnetischen Feldes mit mindestens einer der besagten Retransmitter-Resonanzfrequenzen konfiguriert sind;
ii) Empfängermittel (12;14a,14b), die zum Empfangen eines elektromagnetischen Feldes, das durch die besagten Retransmittermittel retransmittiert wird, konfiguriert sind, wobei die besagten Empfängermittel das besagte elektromagnetische Feld in eine proportionale Spannung umwandeln;
**dadurch gekennzeichnet, dass**:
die besagten Transmittermittel und Empfängermittel in einer bekannten Positions- und Orientierungsbeziehung zueinander gehalten werden, wodurch ein Referenzsystem der besagten Trackingvorrichtung definiert wird, und wobei die besagten elektrischen Schaltungsmittel weiterhin umfassen
iii) Berechnungsmittel, die ausgehend von der besagten, von den Empfängermitteln erhaltenen proportionalen Spannung zum Bestimmen einer Position und Orientierung der besagten Retransmittermittel und damit einhergehend der besagten Trackerstruktur in Bezug auf das besagte Referenzsystem konfiguriert sind, woraus die Position und Orientierung des besagten Objektes verfolgbar ist;
wobei sich die besagten Retransmitter-Resonanzfrequenzen wesentlich von den in der besagten MR-Sequenz erzeugten Frequenzen unterscheiden;
und wobei die besagten Transmittermittel, Retransmittermittel und Empfängermittel von den besagten RF-Transmittermitteln und Kodiermitteln elektromagnetisch entkoppelt sind,
und wobei die besagten Transmittermittel und die besagten Empfängermittel voneinander elektromagnetisch entkoppelt sind,
um ein ununterbrochenes Tracking von Position und Orientierung während des Betriebs der MR-Vorrichtung zu ermöglichen.

2. Trackingsystem nach Anspruch 1, wobei die besagte Trackerstruktur zum Anbringen an einen Kopfbereich eines Patienten konfiguriert ist, wodurch eine feste Position der Retransmittermittel in Bezug auf den besagten Kopfbereich hergestellt wird.

3. Trackingsystem nach Anspruch 2, wobei die besagte Trackerstruktur ausgewählt ist aus der Gruppe bestehend aus Ohrenschützer, Ohrstöpsel, Helm, Kopfgarnitur, Zahnbefestigung, Kieferbefestigung, Nasenklammer, Handgelenkbefestigung, Knöchelbefestigung. stereotaktischer Rahmen, Schiene und Prothese.

4. Trackingsystem nach einem der Ansprüche 1 bis 3, wobei die besagten Transmittermittel und die besagten Empfängermittel fest an einer Strukturkomponente der besagten MR-Vorrichtung angebracht sind.

5. Trackingsystem nach einem der Ansprüche 1 bis 4, wobei die besagten Empfängermittel als Gradiometerschleifenempfänger konfiguriert sind.

6. Trackingsystem nach einem der Ansprüche 1 bis 5, wobei die besagten Retransmitter-Resonanzfrequenzen im Bereich von 50 kHz bis 20 MHz, vorzugsweise im Bereich von 500 kHz bis 5 MHz liegen.

7. Trackingsystem nach einem der Ansprüche 1 bis 6, wobei die besagten Transmittermittel als Transmitterschleife mit einer Transmitterschleifengrösse konfiguriert sind und wobei die besagten Empfängermittel als Empfängerschleifen konfiguriert sind, die eine Empfängerschleifengrösse aufweisen, die wesentlich kleiner ist als die Transmitterschleifengrösse.

8. Trackingsystem nach Anspruch 7, wobei die Empfängerschleifen innerhalb der besagten Transmitterschleife positioniert sind.

9. Trackingsystem nach einem der Ansprüche 1 bis 8, wobei die besagten Retransmittermittel mindestens ein Resonanzschleifenelement umfassen.

10. Trackingsystem nach einem der Ansprüche 1 bis 9, wobei die MR-Vorrichtung eine MR-Bildgebungsvorrichtung ist, wobei die besagte Trackerstruktur mindestens einen MR-aktiven Marker zum Ermitteln der relativen Position und Orientierung des Objekts aus MR-Bildgebungsdaten umfasst, die das Objekt und den aktiven MR-Marker umfassen.

11. Verfahren zum Tracking der Position und Orientierung eines Objekts in einem Trackingsystem nach einem der Ansprüche 1 bis 9, wobei das Verfahren die folgenden Schritte umfasst:
a) festes Anbringen der besagten Trackerstruktur an das Objekt, dessen Position und Orientierung getrackt werden soll;
b) Betreiben der besagten Transmittermittel zum Transmittieren eines elektromagnetischen Feldes mit mindestens einer der besagten Retransmitter-Resonanzfrequenzen;
c) Empfangen eines momentanen elektromagnetischen Feldes, das durch die besagten Retransmittermittel retransmittiert wird; und
d) Berechnen einer momentanen Position und Orientierung der besagten Retransmittermittel, und damit einhergehend der besagten Trackerstruktur in Bezug auf das besagte Referenzsystem, woraus die Position und Ausrichtung des besagten Objektes trackbar ist;

12. Verfahren nach Anspruch 11, wobei die besagte MR-Vorrichtung eine MR-Bildgebungsvorrichtung ist, und wobei die besagte Trackerstruktur mindestens einen MR-aktiven Marker zum Ermitteln der relativen Position und Orientierung des Objekts aus MR-Bildgebungsdaten umfasst, die das Objekt und den aktiven MR-Marker umfassen, wobei das Verfahren weiterhin die Schritte des Erfassens der besagten MR-Bilddaten des Objekts beziehungsweise des aktiven MR-Markers oder der aktiven MR-Marker und des anschliessenden Bestimmens einer relativen Position und/oder Orientierung zwischen dem besagten Objekt und der besagten Trackerstruktur umfasst.

13. Verfahren nach Anspruch 11 oder 12, weiterhin umfassend einen Schritt ausgewählt aus der Gruppe bestehend aus:
- Ausgeben einer Warnung, dass eine Änderung der Position und/oder Orientierung des Objekts stattgefunden hat, die einen vordefinierten Schwellenwert überschreitet;
- Verwerfen und gegebenenfalls Wiederholen der Erfassung eines Satzes von MR-Signalen vom besagten Objekt, wenn eine Änderung der Position und/oder Orientierung des Objekts stattgefunden hat, die einen vordefinierten Schwellenwert überschreitet;
- Verwenden von Positions- und Orientierungsinformationen für die MR-Bildrekonstruktion;
- Anwenden einer Korrektur für Bewegungsartefakte bei der MR-Bildrekonstruktion;
- Aktualisieren der MR-Sequenz;
- Extraktion physiologischer Informationen wie beispielsweise über Zustände, z.B. Tremor oder epileptischer Anfall, und Parameter, z.B. Atmung, Herzschlag, Muskelverspannung oder Nervosität;
- kombinierte Analyse von physiologischen Informationen und MR-Daten.

14. Verfahren nach einem der Ansprüche 11 bis 13, wobei das besagte Objekt ein menschliches Subjekt ist und wobei die besagte Trackerstruktur an einer Stelle des besagten Subjektes ausgewählt aus der Gruppe von: Innenohr, Schädel, Zahn, Kiefer, Nase, Handgelenk und Knöchel angebracht ist.

## Revendications

1. Système de poursuite de position et d'orientation d'un objet dans un appareil à résonance magnétique (MR), le système comprenant un appareil MR et un dispositif de poursuite pour des mesures électromagnétiques de la position et de l'orientation,
l'appareil MR comprenant :
a) un moyen magnétique configuré pour générer un champ magnétique principal dans une région d'échantillon ;
b) un moyen de codage pour générer des champs magnétiques de codage superposés au champ magnétique principal ;
c) un transmetteur FR configuré pour générer des champs de radiofréquences MR ;
d) un moyen pilote configuré pour activer le moyen de codage et le moyen de transmission FR pour générer des champs de codage et des champs de radiofréquences dépendant du temps, superposés, selon une séquence MR pour former des images ou des spectres ; et
e) des moyens d'acquisition pour acquérir un signal MR de ce objet ;
le dispositif de poursuite comprenant :
a) une structure de poursuite (2) qui est fermement attachée à l'objet dont on poursuit la position et l'orientation ;
b) un moyen de retransmission (6 ; 8a ; 8b ; 8c) fixé fermement à la structure de poursuite, ce moyen de retransmission ayant au moins une fréquence de résonance de retransmission ; et
c) un circuit électronique comprenant :
i) un moyen de transmission (10) configuré pour transmettre un champ électromagnétique à au moins une des fréquences des résonance de retransmission,
ii) un moyen de réception (12 ; 14a ; 14b) configuré pour recevoir un champ électromagnétique retransmis par le moyen de retransmission ; ce moyen récepteur convertissant le champ électromagnétique en une tension proportionnelle,
système **caractérisé en ce que**
le moyen de transmission et le moyen de réception sont maintenus dans une relation de position et d'orientation connue, l'un à l'autre, définissant ainsi un cadre de référence du dispositif de poursuite ; et
- le circuit électrique comprenant en outre :
iii) un moyen de calcul configuré pour déterminer à partir de la tension proportionnelle reçue du moyen de réception, une position et orientation du moyen de retransmission concomitante avec la structure de poursuite par rapport au cadre de référence à partir desquelles on peut tracer la position et l'orientation de l'objet,
- les fréquences de résonance de retransmission étant substantiellement différentes des fréquences générées dans la séquence MR ;
et les moyens de transmission, les moyens de retransmission et les moyens de réception étant découplés électromagnétiquement des moyens de transmission FR et des moyens de codage, et les moyens de transmission et les moyens de réception étant découplés électromagnétiquement l'un de l'autre de façon à permettre une poursuite ininterrompue de position et l'orientation pendant le fonctionnement de l'appareil MR.

2. Système de poursuite selon la revendication 1,
dans lequel
la structure de poursuite est configurée pour être fixée à un région de tête d'un patient, la position fixée du moyen de retransmission étant établie en relation avec la région de tête.

3. Système de poursuite selon la revendication 2,
dans lequel
la structure de poursuite est choisie dans le groupe comprenant : un cache-oreille, un bouchon à oreille, un casque, un couvre-chef, une fixation de dent, une fixation de mâchoire, une boucle de nez, une fixation de poignet, une fixation de cheville, un cadre stéréo tactique, une attelle et une prothèse.

4. Système de poursuite selon l'une des revendications 1 à 3,
dans lequel
les moyens de transmission et les moyens de réception sont fermement fixés à un composant de structure de l'appareil MR.

5. Système de poursuite selon l'une des revendications 1 à 4,
dans lequel
les moyens de réception sont configurés comme récepteurs de boucle de gradiomètre.

6. Système de poursuite selon l'une des revendications 1 à 5,
dans lequel
les fréquences de résonance de retransmission sont dans la plage de 50 KHz-20 MHz, de préférence dans la plage de 500 kHz à 5 MHz.

7. Système de poursuite selon l'une des revendications 1 à 6,
dans lequel
les moyens de transmission sont configurés comme boucles de transmission avec une taille de boucle de transmission, et
les moyens de réception sont configurés comme boucles de réception ayant une taille de boucle de réception qui est substantiellement inférieure à la taille de boucle de transmission.

8. Système de poursuite selon la revendication 7,
dans lequel
les boucles de réception sont placées dans la boucle de transmission.

9. Système de poursuite selon l'une des revendications 1 à 8,
dans lequel
les moyens de retransmission comprenant au moins un élément de boucle résonant.

10. Système de poursuite selon l'une des revendications 1 à 9,
dans lequel
l'appareil MR est un appareil d'imagerie MR, la structure de poursuite comprenant au moins un marqueur actif MR pour établir la position et l'orientation relatives de l'objet à partir des données d'imagerie MR comprenant l'objet et le marqueur MR actif.

11. Procédé de poursuite de position et d'orientation d'un objet dans un système de poursuite selon l'une des revendications 1 à 9,
le procédé comprenant les étapes consistant à :
a) fixer fermement la structure de poursuite à l' objet dont on poursuit la position et l'orientation ;
b) actionner le moyen de transmission pour transmettre le champ électromagnétique à au moins l'une des fréquences de résonance de transmission ;
c) recevoir le champ électromagnétique instantané retransmis par le moyen de retransmission ; et
d) calculer la position et l'orientation instantanées du moyen de retransmission et de façon concomitante, de la structure de poursuite par rapport à la trame de référence et dont on poursuit la position et l'orientation de l'objet.

12. Procédé selon la revendication 11,
selon lequel
l'appareil MR est un appareil d'imagerie MR, et
la structure de poursuite comprend au moins un marqueur MR actif pour établir la position et l'orientation relatives de l'objet à partir des données d'imagerie MR comprenant l'objet le marqueur MR actif,
procédé comprenant en outre
les étapes d'acquisition des données d'image MR de l'objet et le ou les marqueurs MR actifs respectifs, et ensuite déterminer à partir de là, une position et/ou orientation relatives entre l'objet et la structure de poursuite.

13. Procédé selon la revendication 11 ou 12,
comprenant en outre
une étape choisie dans le groupe comprenant :
- émettre un avertissement indiquant qu'il y a eu un changement de position et/ou d'orientation de l'objet dépassant un seuil prédéfini ;
- rejeter et en option, répéter l'acquisition d'un jeu de signaux MR de l'objet lorsqu'il y a eu un changement de position et/ou d'orientation de l'objet dépassant un seuil prédéfini ;
- utiliser l'information de position et d'orientation pour la reconstruction de l'image MR ;
- appliquer une correction aux artifices de mouvement de reconstruction d'image MR ;
- mettre à jour la séquence MR ;
- extraire une information physiologique telle que pour les états en général le tremblement ou une crise épileptique et les paramètres concernant en général la respiration, le rythme cardiaque, la tension musculaire ou la nervosité ;
- l'analyse combinée de l'information physiologique et des données MR.

14. Procédé selon l'une des revendications 11 à 13,
selon lequel
l'objet est un sujet humain, et
la structure de poursuite est fixée à un emplacement du sujet, choisi dans le groupe, comprenant : oreille interne, squelette, dent, mâchoire, nez, poignet et cheville.
